(19) Europäisches Patentamt
European Patent Office
Office européen des brevets

(11) EP 1 923 808 A2

(12) **EUROPEAN PATENT APPLICATION**

(43) Date of publication:
**21.05.2008 Bulletin 2008/21**

(51) Int Cl.:
***G06F 19/00*** *(2006.01)*

(21) Application number: **07114077.6**

(22) Date of filing: **09.08.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **08.09.2006 EP 06018856**

(71) Applicant: **F.HOFFMANN-LA ROCHE AG**
**4070 Basel (CH)**

(72) Inventor: **Laczko, Endre**
**Basel 4054 (CH)**

(74) Representative: **Vossius & Partner**
**Siebertstrasse 4**
**81675 München (DE)**

(54) **Method for predicting biological, biochemical, biophysical, or pharmacological characteristics of a substance**

(57) The method for predicting biological, biochemical, biophysical, or pharmacological characteristics of a substance comprises the steps of providing sample data, scaling the sample data by coding the sample data, classifying the coded data, and providing a prediction output on the basis of the classification result.

**Fig. 1**

| **Sample analysis** | **Data analysis** | | | |
|---|---|---|---|---|
| Measurement and basic data transformation (FID to spectra, m/z records to chromatograms and mass spectra) | Data pre-processing = data coding | | | Numerical analysis |
| Delivery of primary data → | Alignment of signals or binning | Normalize data sample wise to unit integral = convert primary data into profiles | Replace missing values by median of reference values, re-code data into deviation profiles with ordinal scale **= scaling by IQRHILO coding** | Model and classify all profiles coded as deviation profiles, predict from deviation profile type |
| Assure analytical quality | Assure equity of recorded signals | Eliminate global elements of confounding variability (shared variability affecting all samples | Isolate significant treatment related differences between groups (samples) of interest, eliminate confounding individual or time dependent parts of normal variability | Assign meaning to the changes, interpret in the context of the original research question |

EP 1 923 808 A2

Printed by Jouve, 75001 PARIS (FR)

## Description

Field of the Invention

The present invention provides a method for predicting biological, biochemical, biophysical, or pharmacological characteristics of a substance. In particular, the invention provides for predicting toxicity of a substance.

Background of the Invention

In today's biological research, 'omics' technologies (e.g. metabonomics, toxicogenomics) are widely used to investigate changes on the molecular and biochemical level in relation to an experimental perturbation. To enable the detection and numerical analysis (NA) of these perturbation related changes, primary 'omics' data are typically transformed by various pre-processing or coding procedures into equally scaled profiles. However, prior art coding procedures apply only to specific experimental designs and to only one of the 'omics' techniques. If nowadays new and combined applications are considered, new and tailored coding procedures are required too. The use of 'omics' in the context of drug development is concerned with two important problems: (i) seen from an applied perspective, there is no coding optimized for realistic perturbation experiments with restricted samplings and replicates, and (ii) more general, there is a need for a coding procedure that enables the combined NA of 'omics' profiles and other data types.

Summary of the Invention

The invention relates to data coding and integration, for example for the numerical analysis of 'omics' profiles. The invention provides a method for predicting biological, biochemical, biophysical, or pharmacological characteristics of a substance, for example toxicity of a substance. The method preferably comprises the steps of: (a) providing sample data; (b) scaling the sample data by coding the sample data; (c) classifying the coded data; and (d) providing a prediction output on the basis of the classification result.

The step of providing sample data is preferably followed by the step (a1) of binning of the provided data.

The data are preferably normalized sample wise to unit integral in order to convert the data into sample profiles. The sample profiles are, for example, nuclear magnetic resonance (NMR) profiles, and the profiles are preferably in the form of NMR spectral data.

According to the method of the invention, the spectral data are preferably arranged to form a matrix. The data are arranged in the matrix preferably such that all bin values for a given spectrum are in one row and all values for a given bin or spectral region are in one column.

According to a preferred embodiment, the method comprises further the step of arranging one or more sample descriptors in one or more auxiliary column vectors of the same length and order as the column vectors of the data matrix. The one or more sample descriptors are, for example, selected from the group comprising study number, animal number, sampling time, dose group, toxicity class, toxicological variables.

In a further preferred step, missing data values in the matrix are replaced. The missing data are, for example, formed by the median of reference values. In more detail, the missing data are formed by the median of the corresponding control group at the corresponding sampling time.

Step (c) of coding the data preferably comprises replacing all values of the matrix in accordance with a given rule. More preferably, the values of the matrix are replaced by ordinal values indicating the order of deviation from a reference region. The replacement is, for example, made as n-level ordinal scaling. More preferably, a 3-level ordinal scaling is used, and the values of the matrix were replaced by 0, 1, or 2, depending on whether the value was below, in, or above the range defined by the $x^{th}$ and $y^{th}$ inter-quantile range of a corresponding control group. Herein, x is for example 10, and y is for example 90. Alternatively, binary scaling is used.

Step (c) preferably comprises the step of assigning the individual coded data to groups. The groups preferably correspond to specific dosing levels of a specific compound. In step (d) the predictive output is generated for at least one group.

The method and its preferred steps will no be described in more detail.

*Data and toxicity classes*

Table 1 shown below lists the 22 COMET1 studies referenced by the NMR spectra used in the discussed examples (the COMET1 project is described in Lindon, J.C. et al. Contemporary issues in toxicology - The role of metabonomics in toxicology and its evaluation by the COMET project. Toxicology and Applied Pharmacology 187, 137-146 (2003)). An artificial set of normal profiles (all profile values coded to 1, meaning no deviation from reference) was added to the 21 COMET1 studies, thus the test set comprised in total 22 well defined profile groups. Assumed organ toxicity classes and main toxicity targets are indicated. The following number codes were used for the organ toxicity classes (Tox class): 0 = non toxic, 1 = other (pancreatic, lymphoid, immunotoxic, testicular, peroxisome proliferator), 2 = hepatic, 3 = nephretic, 7 = hepatic and Hydrazine like, 9 = hepatic and peroxisome proliferation activator. A refinement of the principal toxicity and the diagnosis in relation to the expected target and mechanism were added. R12 was the only study performed with HanWistar rats, all other studies were performed with SpragueDawley rats (Crl:CD (SD)IGS BR). Furthermore, the following abbreviations are used : (cc = clinical chemistry, "-" = signs of toxicity absent, "+" = signs of toxicity observed, histo = histopathology, prolif. = proliferation, reg = regeneration, rco = renal compensation, sub = subtoxic, only minimal to mild reversible changes, bil = biliary, r. reaction)

**Table 1**

| Study No | Treatment | Tox class | Indication | Main target | Subclass / 2$^{nd}$ toxicity | Diagnosis | |
|---|---|---|---|---|---|---|---|
| | | | | | | histo | cc |
| CTRL | Artificial normal profiles | ntx | Non Toxic | Normal tissue | None | 0 | - |
| D05 | Partial hepatectomy | ntx | NonToxic | Liver | Regeneration | 2reg | + |
| D06 | Unilat. nephrectomy | ntx | NonToxic | Kidney | Compensation | 0rco | + |
| D07 | Gentamicin | k | Toxin | Nephrotoxic | Proximal tubules | 3sub | + |
| L01 | Hydrazine | l | Toxicant | Hepatotoxic | Steatosis | 7 | + |
| L05 | Clofibrate | o | Toxicant | Hepatotoxic | Peroxisome prolif. | 9sub | + |
| L07 | Mercury Chloride | k | Toxicant | Nephrotoxic | Proximal tubules | 3 | + |
| L12 | Deprevation of food | ntx | NonToxic | Starvation | Thymus, liver | 0 | + |
| L13 | Deprevation of water | ntx | NonToxic | Dehydration | Kidney | 0 | + |
| L14 | Vancomycin | k | Toxicant | Nephrotoxic | Proximal tubules | 3reg | + |
| L16 | Maleic acid | k | Toxicant | Nephrotoxic | Proximal tubules | 3 | + |
| N02 | ANIT | l | Toxicant | Hepatotoxic | Direct r. & biliary | 2bil | + |
| N09 | N-methylformamid | l | Toxicant | Hepatotoxic | Direct reaction | 2 | + |
| N14 | Di-methylnitrosamin | l | Toxicant | Hepatotoxic | Direct reaction | 2 | + |
| R01 | Hydrazine | l | Toxicant | Hepatotoxic | Steatosis | 7 | + |
| R09 | Acetazolamide | k | Toxicant | Nephrotoxic | Papilla | 3sub | - |
| R12 | Hydrazine | l | Toxicant | Hepatotoxic | Steatosis | 7 | + |
| S01 | Hydrazine | l | Toxicant | Hepatotoxic | Steatosis | 7 | + |
| S04 | Ammonium Chloride | ntx | NonToxic | Kidney | Metabolic acidosis | 0 | + |
| S06 | Mitomycin | o | Toxin | Nephrotoxic | Tubules, blood | 1hae 3 | + |
| S08 | Methotrexate | o | Toxicant | Hemotoxic | Liver & kidney | 1hea | - |
| S11 | Sodiumbicarbonate | ntx | NonToxic | Kidney | Alkalosis | 0 | + |

Briefly, in these studies urine samples from male Sprague Dawley rats were collected between - 24h pre and 168h post dose in 8, 16 or 24h intervals. In each experiment 10 replicate animals were used as controls and at each treatment level. In the examples described herein in the following, only the urinary metabonomic profiles of the 24h to 48h urine collection from controls and the highest dose level group were used. The spectra were selected and grouped from the 22 studies according to the observed pathology at 48h post dose. A balanced data set was achieved by grouping of the studies and profiles according to the toxicity classes non-toxic (class code ntx), liver (class code 1), kidney (class code k) and others (class code o), whereby others include liver peroxisome proliferation, testis, thymus, blood and pancreas. If appropriate, cases with observed tissue regeneration (subclass code r), with slight to mild tissue lesions (subclass code s) and overt tissue lesions (no subclass code) were differentiated within these classes.

It should be understood that the use of these specific data is by way of example only in order to facilitate understanding of the present invention, and should not be considered as limiting the invention in any way.

*Excluded Spectral regions (bins)*

The COMET1 NMR profiles are preferably initially in the form of water region excluded, binned and normalized (i.e., scaled to unit integral) NMR spectra with 205 bins of 0.04 ppm width covering a spectral range from 9.96 ppm to 0.24 ppm. Furthermore, spectral regions (bins) related to excreted native drug or its metabolites (drug related compounds or DRCs), are also preferably excluded from these spectra (see Ebbels, T. M., H. Keun, et al. (2003). "Toxicitiy classification from metabonomic data using a density superposition approach: "CLOUDS"." Analytica Chimica Acta 490: 109-122). However, these profiles still contain bins known to be affected by activity or compositional changes of the gut microflora. These bins confound the interpretation of treatment related effects and are not considered in any data analysis or prediction procedures. Also, the bins at the extremes of the stored spectra profiles are preferably excluded from analysis and prediction because of their variability seen in previous explorative data analysis.

*Definition of data matrices and vectors used for analysis and prediction*

In a first step, the spectral data of interest are preferably re-arranged to form a matrix X with all bin values for a given spectra in one row and with all values for a given bin or spectral region in one column:

$$\downarrow c_1 \qquad \rightarrow r_1$$

$$X = \begin{vmatrix} x_{1,1} & x_{1,2} & \cdots & x_{1,p} \\ x_{2,1} & x_{2,2} & \cdots & x_{2,p} \\ \vdots & \vdots & & \vdots \\ x_{n,1} & x_{n,2} & \cdots & x_{n,p} \end{vmatrix}$$

Thus, row vectors $\rightarrow r$ represent one binned spectra characterizing one sample, with

$$\begin{aligned} &\text{binned spectra(sample 1)} \rightarrow r_1 = (x_{1,1}, x_{1,2}, \ldots, x_{1,p}) \\ &\text{binned spectra(sample 2)} \rightarrow r_2 = (x_{2,1}, x_{2,2}, \ldots, x_{2,p}) \\ &\quad \vdots \\ &\text{binned spectra(sample n)} \rightarrow r_n = (x_{n,1}, x_{n,2}, \ldots, x_{n,p}), \end{aligned}$$

and column vectors $\downarrow c$ represent the variability of a fixed spectral region or bin among the samples included in the matrix, with

$$\begin{aligned} &\text{bin 1(samples 1 to n)} \downarrow c_1 = (x_{1,1}, x_{2,1}, \ldots, x_{n,1}) \\ &\text{bin 2(samples 1 to n)} \downarrow c_2 = (x_{1,2}, x_{2,2}, \ldots, x_{n,2}) \\ &\quad \vdots \\ &\text{bin p(samples 1 to n)} \downarrow c_p = (x_{1,p}, x_{2,p}, \ldots, x_{n,p}). \end{aligned}$$

Sample descriptors, notably study number, animal number, sampling time, dose group, toxicity class and toxicological variables, are preferably arranged in auxiliary column vectors $\downarrow ac$ of the same length and order as vectors $\downarrow c$.

$$\text{study number(samples 1 to n)} \downarrow ac_{sno} = (sno_1, \ldots, sno_n)$$
$$\text{animal number(samples 1 to n)} \downarrow ac_{ano} = (ano_1, \ldots, ano_n)$$
$$\text{sampling time(samples 1 to n)} \downarrow ac_{stime} = (stime_1, \ldots, stime_n)$$
$$\text{dose group(samples 1 to n)} \downarrow ac_{dgr} = (dgr_1, \ldots, dgr_n)$$
$$\text{toxicity class(samples 1 to n)} \downarrow ac_{class} = (y_1, \ldots, y_n)$$
$$\text{toxicological variable1(samples 1 to n)} = (v1_1, \ldots, v1_n)$$

These auxiliary vectors are used either to sort spectra or, if appropriate, as dependent variable y or matrix Y to be modelled by X in the sense of a prediction or an estimation.

*Replacement of missing values*

The spectral data of matrix X are preferably additionally pre-processed. For example, in a first step, all bins (column vectors) are checked for missing values. Detected missing values in matrix X and if appropriate in Y, are preferably replaced by the median of the corresponding control group at the corresponding sampling time. In more detail, this is equal to look up the study number and sampling time attributed to a numerical analysis (NA), then to generate a sublist with the values of the same bin attributed to the animals in the control group of the same study and sampling time, eventually to determine the median value in this sublist and to replace NA. This replacement procedure can be written more formally as

$$\text{NA in } bin_a \text{ for } sno=sno_i,\ stime=stime_l,\ ano=ano_k \text{ <- replaced by}$$
$$\text{median of sublist in } bin_a \text{ for } sno=sno_i,\ stime = stime_l,\ dgr = CTRL.$$

This replacement is preferred because it is neutral in respect of the following pre-processing steps in the sense that the added information (the values replacing the missing values) does not amount to an artificial deviation from the control group.

*Recoding of data matrix X by IQRHILO coding (IQRHILO = inter-quantile range high low)*

In a second step, all values of the data matrix X are replaced by 0, 1 or 2, depending whether the value is below, in or above the range defined by 10th and 90th quantile (inter-quantile range or IQR) of the corresponding control group. Thus,

$$x \text{ in } bin_a \text{ for } sno=sno_i,\ stime=stime_l,\ ano=ano_k = \begin{cases} 2 & x > q(90)_{CTRL} \\ 1 & \text{otherwise} \\ 0 & x < q(10)_{CTRL} \end{cases}$$

with

$$q(..)_{CTRL} = (..)\text{th quantile of sublist in } bin_a \text{ for } sno=sno_i,\ stime = stime_l,\ dgr = CTRL.$$

In contrast to, for example, piecewise linear coding (fuzzy coding), the IQRHILO coding-strategy according to the invention neutralizes variability within the inter-quantile range (IQR) and emphasizes extra-IQR variability. This corresponds better with the objectives of data analysis in the context of toxicity testing and prediction. IQRHILO coding integrates filtering and scaling by a time and condition matched reference range, whereby the reference range and scale could be freely defined. The present IQRHILO coding is thus comparable to a deviation rating. It keeps for all values of the spectral data matrix X the information that a deviation from the reference range occurred (if $x_{replaced} \neq 1$) or not (if

$x_{replaced}$ = 1) as well as the deviation's direction (increased if $x_{replaced}$ = 2, decreased if $x_{replaced}$ = 0). This amounts to an equal weighting of all spectral bins, thus to a representation of spectral deviation that depends only on the number and direction of deviating bins in a spectra and not on the magnitude of individual bin deviations. IQRHILO coding according to the invention is considered to increase the sensitivity for small and "short living" overall spectral deviations, while keeping the risk of converting spectral noise or outliers into misleading indicators of deviation on a minimum (loss of specificity and variance stability), hence to enhance spectra based clustering, classification as well as prediction and, finally, to enable prediction based on single urine samplings. Additionally, the recoded matrix $X_{IQRHILO}$ might be used to draw a heat map of the spectral changes sorted by the auxiliary column vectors and the bins.

*CLOUDS classifier*

Finally, the present invention preferably uses the CLOUDS classifier (see Ebbels, T. M., H. Keun, et al.) as developed within the COMET project (see Lindon, J.C. et al.). The implementation of the CLOUDS classifier requires an assignment of individual spectra to a group. The predictive output is generated for the group only, normally for a specific compound and dosing level, and not for individual replicate spectra. More precisely, the predictive output for each group is a list of n values indicating the similarity between the test group and each of the n reference groups of the training set used to calibrate the classifier. This list allows ranking of the reference groups, representing specific compounds and dosing levels and ultimately an associated toxicity class, in an order of ascending similarity. The test group has eventually to be classified according to defined rules to a toxicity class represented by the most similar reference groups. The preferred rules of prediction are:

Classify test group to the class of the most similar study or as non-toxic if one of the following statements applies:

first hit similarity <0.5
first hit corresponds to class 0
first hit corresponds to a study with sub toxic outcome
first hit corresponds to a study with regeneration only which itself classifies to a study of class 0

Consider following hits for interpretation, including assessment of prediction quality.

Brief description of the drawings

The invention will now be described in more detail with respect to the accompanying Figures, in which

Fig. 1 provides a schematic overview of the method of the invention and how it is embedded in pre-processing steps; and

Fig. 2 shows correspondence analysis of binned-normalized spectra and of binned-normalized-IQRIDLO coded profiles.

Detailed description of the Invention

The present invention introduces interquantile range (IQR) coding, a novel data coding method conceived to overcome the disadvantages of known methods as outlined in the Background section above. The inventors tested IQR coding first with urinary metabonomic profiles and other toxicological data from the COMET1 project (see Lindon, J.C.). IQR coding was then applied to data from other initial preclinical toxicity studies with rats. Typically, in these latter studies urine, serum, and tissue samples were collected from 5 to 10 individuals per dosing level, including controls, within 48h after a single dose. The resulting data included metabonomic profiles based on urinary 1H NMR spectra, clinical chemistry and histopathology assessments. IQR coding was used prior to multivariate analysis and metabonomics based toxicity classification.

Fig. 1 shows a summary and positioning of data pre-processing by IQR coding according to a preferred embodiment of the present invention. On top the main steps from data acquisition to interpretation are indicated. In the midsection a typical flow of pre-processing steps, including IQR coding of the present invention, is detailed. At the bottom the objectives of individual steps are given. The annotated workflow reflects the principal steps and objectives of a common 'omics' data analysis scheme. This scheme exemplifies one possible application of IQR coding on metabonomics profiles. It should be noted that IQR coding in this preferred embodiment is combined with other commonly performed data pre-processing steps, i.e. in the illustrated case data reduction by binning (bucketing) and normalization to unit sum. The shown combination is not meant to be exclusive but may be changed, shortened or extended, e.g. if primary data were generated on other technology platforms and characterize other entities. It can be seen that data pre-processing follows sample analysis and basic transformation of measurements into raw data. This is a multi step processing of raw data to enable proper data interpretation in the context of a specific question and is part of data analysis. Data pre-processing by IQRHILO coding according to the invention, as well as NA-to-median replacement may be applied to raw data or combined with any (likely preceding) quality measures (e.g. peak matching, binning) and global data scaling (e.g. scaling to unit integral). IQRHILO coding is a one step procedure to isolate significant treatment related differences between groups (samples) of interest by re-coding data into deviation profiles and is effective in the frame of a toxicological experiment or survey. Both support toxicological or pharmacological objectives and not only 'omics' but all kind of data.

As indicated in Fig. 1, IQR coding transforms primary data or profiles (i.e. in the illustrated example reduced and normalized spectra) into deviation profiles giving for each sample in each variable an ordinal deviation value (i.e. an ordinal fold change) or a binary deviation indicator (i.e. deviation observed or not observed) in reference to appropriate control values. Unique to the concept of IQR coding are the procedures to identify and to estimate deviations and, implicitly connected to this, the definition of the appropriate reference. As the term "IQR coding" suggests, deviations were defined as values outside a reference interquantile range. The $10^{th}$ and $90^{th}$ percentiles of the appropriate control range are preferably chosen to specify the reference IQR. The control values belonging to exactly the same experimental condition, i.e. study, mode of treatment, sampling time (age, exposure to experimental factors), generation and genetic line are defined as the appropriate control values, or reference. This means in the frame of typical data sets with repeated samplings and 10 replicates in the control group that for each sampling instance its time matched reference IQR is calculated, whereby the highest and the lowest control values are not used. This specification of the IQR may be easily adapted for other data sets or objectives by choosing different limiting percentiles as well as a different definition of the appropriate reference condition. Finally, all values are replaced by ordinal values indicating the order to deviation from the reference IQR. For example, all values of the data are replaced with 0, 1 or 2 to code abnormal decrease (0), deviation within normal range (1) or abnormal increase (2). This variant with a 3 level ordinal scaling is regarded as IQRHILO coding according to the invention. Preferably, one can select easily finer or coarser granularities of the ordinal scale. For example, a binary scale is encompassed, which is especially useful to condense data groupwise and to calculate variablewise incidence rates and incidence statistics.

IQR coding according to the invention is more in accordance with toxicological practice than other data pre-processing procedures because it filters out confounding variance related to normal inter-individual variability (i.e. variability within and across generations and genetic lines), development and continued adaptation. It should be noted that IQR coding does not require the exclusion of extremes or deviating responses in controls, but can treat them as potentially interpretable features. This is because it codes deviation from the normal interquantile range as a binary or ordinal value, emphasizing the quality and not the exact magnitude of a deviation. By this, IQR coding also opens comprehensible strategies to numerically integrate data from different sources and technologies (as ordinal deviation or incidence rate) as well as the possibility to numerically harmonize data of heterogeneous quality to the highest shared precision (finest granularity of a shared ordinal scale) or useful precision. Since the recognition and enumeration of significantly increased and decreased variables constitute most often the only biologically interpretable part of an NAA, IQRHILO coding is most often compliant with the highest useful precision.

From a statistical point of view, IQR coding may be seen as an optimized robust significance filter which can be used in place of other recently suggested, less robust or less sensitive methods. Furthermore, IQR coding is an alternative way to avoid difficulties (e.g. spurious correlations, absence of an interpretable covariance structure) associated with the analysis of multivariate compositional data as normalized 'omics' profiles usually are. And last, IQR coding weighs all variable changes equally. The overall effect is that after IQR coding, profiles with changes in similar subgroups of variables (i.e. high overlap in number and identify of changed variables) are identified as being associated, or in other words, being members of the same cluster. These characteristics let us argue that IQR coding, if applied in the frame of any perturbation experiment, extracts perturbation related information and facilitates NA, i.e. un-supervised as well as supervised multivariate data analysis. This is supported with two examples of metabonomics based toxicity modeling, which build on experiments where a test compound is administered to cause perturbation of metabolism.

Example 1

The first example contrasts an un-supervised correspondence analysis (CA) of binned-normalized and binned-normalized-IQRHILO coded spectral profiles (see Fig. 2). The purpose of CA is to visualize how far the hidden patterns in the data set correspond with pre-defined sample groups or classes. CA is chosen because it can be used to map the sample similarity and the sample-variable association on the base of a $\chi^2$-metric which is equally appropriate for the binned-normalized and binned-normalized-IQRHILO coded data. This enables the proper comparison of the data set's structure (the proximity of samples) after the two different pre-processings on an equal scale. The resulting maps in the plane of the first two CA axes are shown in Fig. 2. It is concluded that IQRHILO coding emphasizes small deviations from controls. Further, it is noted that after IQRHILO coding the distance of most metabonomic parameters to the controls is in accordance with the observed severity of histological lesions. Thus, the geometrical distance to controls has a toxicological meaning. This supports the claimed toxicological relevance of IQRHILO coding. In summary, IQRHILO coding seems to enhance discrimination and identification of sample groups representing different toxicities (or perturbations) as well as different grades of toxicity (or perturbation).

Fig. 2 shows correspondence analysis of binned-normalized spectra (left map in Fig. 2, F1-F2 display 62.1% of total variance) and of binned-norxnalized-IQRHILO coded profiles (left map in Fig. 2, F1-F2 display 30.5% of total variance). The maps are drawn with the row scores only. The analyzed profiles represent 1H NMR spectra of 215 urine samples, collected 24h to 48h after dosing from 10 male Sprague Dawley rats in 22 toxicity studies with well known compounds. The green circles and the letters CTRL indicate the position of the controls. Colors code toxicity classes: green = controls and non toxic, black = kidney, red = liver, yellow = subtoxic kidney, grey = subtoxic liver, magenta = regeneration, cyan blue = renal compensation, dark blue = other (pancreas, thymus, blood), orange = subtoxic other. Labels indicate the COMET 1 study number.

To help visual comparison, two arbitrary axes, crossing at the coordinates of the controls, are added to the maps. IQRHILO coding isolates the controls and increases the overall spread of sample scores. Within groups of samples the inverse, i.e. better sphering, may be seen (e.g. L07, L05, S01, R01). With a few exceptions (e.g. N15, L05), the relative positions (proximities) of the samples persist (e.g. S01, R01, L01, R12, L07, S11, S04). IQRHILO coding emphasizes small deviations from controls while maintaining and stabilizing the overall dispersion. Most importantly, after IQRHILO coding the distance of most samples to the controls is in accordance with the observed severity of histological lesions (e.g. L05 and N02 with no or minimal liver lesions close to controls, N14 with marked liver lesions more distant from control). Thus, after IQRHILO coding, the geometrical distance to controls has a toxicological meaning: the greater the distance is the more perturbed or toxic is the represented outcome.

<u>Example 2</u>

The second example shows how the above demonstrated advantages translate into enhanced predictive performance. As an example, linear discriminant (LD), k-nearest neighbour (KNN) and supported vector machines (SVM) classifiers as implemented in the statistical software environment R (Venables, W.N. & Ripley, B.D. Modern Applied Statistics with S, Edn. Fourth Edition. (Springer, New York; 2002)) and the CLOUDS classifier (i.e. a modified probabilistic neuronal network classifier) with the sample set used in the first example (previous paragraphs) were tested. First, the predictive performances of LD, KNN and SVM classifiers using either binned-normalized or binned-normalized-IQRHILO coded spectra were contrasted for a 4-class model and for a refined 9-class model, as shown in the following table:

**Table 2**

| Classifier | 4-class model | | 9-class model | |
|---|---|---|---|---|
| | b-n profiles | IQRHILO profiles | b-n profiles | IQRHILO profiles |
| LD | 13.0 | 23.3 | 14.4 | 28.8 |
| KNN | 10.2 | 6.1 | 14.9 | **8.8** |
| SVM | 8.8 | **4.2** | 18.1 | 9.8 |

Table 2 shows Prediction error rates (%) for 4- and 9-class toxicity models indicating the percentage of spectra misclassified (false positives + false negatives) by linear discriminant (LD), k-nearest neighbour (KNN) and supported vector machines (SVM) classifiers, calculated after 9-fold cross validation. The 4-class model discriminates non-toxic, liver toxicity, kidney toxicity and other toxicity, whereas the 9-class model discriminates additionally the subclasses regeneration liver, regeneration kidney, subtoxic changes liver, subtoxic changes kidney and subtoxic changes other tissues. Either binned-normalized (b-n) or binned-normalized-IQRHILO (IQRHILO) coded spectral profiles of the same sample set were used. The profiles represent in all cases 1H NMR spectra of 215 urine samples, collected 24h to 48h after dosing from 10 male Sprague Dawley rats in 22 toxicity studies with well-known compounds.

Thus, IQRHILO coding enhanced the performance of the KNN classifier by 40% and of the SVM classifier by 52% but reduced the performance of the LD classifier. The lowest error rate (4.2%) was achieved with IQRHILO coded data and the SVM classifier. As one might expect, IQRHILO coded data is less adequate for the LD classifier. In principle, the conclusions drawn from the 4-class model also apply for the 9-class model. However increasing the class number reduces the predictive performance. This reduction was less severe in the case of the KNN classifier, resulting in an inversed performance rank of the KNN (8.8%) and SVM (9.8%) classifiers. The same trends were noticed (not shown) for the transition to more extended and complex data sets as well as for the transition to an 11-class model (9-class model of Table 1 with "liver and kidney toxicity" and "subtoxic liver and kidney" added). In a second independent evaluation the inventors contrasted the performance of the CLOUDS classifier using either binned-normalized or binned-normalized-IQRHILO coded spectra for the 4-class model detailed above. Error rates indicating the percentage of misclassified groups (false positives + false negatives) were calculated after groupwise leave one out cross validation, whereby a group was defined by 10 replicates of a single study and condition (i.e. same compound, same dose level, only spectra of samples taken 48h post dose). An error rate of 27% with binned-normalized spectra and of 14% with additional IQRHILO coding was achieved. In summary, using nonlinear and non-parametric classifiers like KNN, SVM and CLOUDS, IQRHILO coding makes it possible to predict five organ toxicity classes and two seventy levels with low error rates of 9 to 14%, even under the restriction of a single sampling occasion. To the inventors knowledge, no other pre-processing of data was reported to be similarly effective and reliable in the frame of pre-clinical toxicity testing of drugs. Within toxicology, similar performance has only been reported for classifiers based on less restricted data sets with rather dense time series of samples or for classifiers based on two class models.

As mentioned above, IQR coding is also a way to integrate data from different sources and technologies. It shall be noted without exemplification that the inventors practiced also IQR coding of histopathology, serum chemistry, microarray and proteomics data for combined NA, and that the statistical correspondence of histopathological grading and serum chemistry, differentially expressed genes, urinary proteins and metabolites by CCA, CVA and CDA was tested. In other words, IQR coding was used to prepare data for multi-table analyses which are well suited to quantify associations between hypothesized explanatory and response variables related to same objects.

IQR or IQRHILO coding is applicable wherever perturbed states of biological systems are to be differentiated and explained by heterogeneous data collections in reference to controls, differentiating perturbation responses from normal (e.g. inter-individual) variability, development and adaptation (growth, changing environmental conditions, rhythms).

## Claims

**1.** Method for predicting biological, biochemical, biophysical, or pharmacological characteristics of a substance, comprising the steps of:

a) providing sample data;
b) scaling the sample data by coding the sample data;
c) classifying the coded data; and
d) providing a prediction output on the basis of the classification result.

**2.** Method of claim 1, further comprising, following step a), the step a1) of binning of the provided data.

**3.** Method of claim 1 or 2, further comprising, following step a) or a1), the step of normalizing the data.

**4.** Method of any of claim 3, wherein the data are normalized sample wise to unit integral in order to convert the data into sample profiles.

**5.** Method of claim 4, wherein the sample profiles are nuclear magnetic resonance (NMR) profiles.

**6.** Method of claim 5, wherein the NMR profiles are in the form of NMR spectral data.

**7.** Method of claim 6, wherein the spectral data are arranged to form a matrix.

**8.** Method of claim 7, wherein the data are arranged in the matrix such that all bin values for a given spectrum are in one row and all values for a given bin or spectral region are in one column.

**9.** Method of claim 8, further comprising the step of arranging one or more sample descriptors in one or more auxiliary column vectors of the same length and order as the column vectors of the data matrix.

**10.** Method of claim 9, wherein the one or more sample descriptors are selected from the group comprising study number, animal number, sampling time, dose group, toxicity class, toxicological variables.

**11.** Method of any of claims 7 to 10, further comprising the step of replacing missing data values in the matrix.

**12.** Method of claim 12, wherein missing data are formed by the median of reference values.

**13.** Method of claim 13, wherein missing data are formed by the median of the corresponding control group at the corresponding sampling time.

**14.** Method of any of claims 7 to 13, wherein the step c) of coding the data comprises replacing all values of the matrix in accordance with a given rule.

**15.** The method of claim 14, wherein the values of the matrix are replaced by ordinal values indicating the order of deviation from a reference region.

**16.** Method of claim 15, wherein the replacement is made as n-level ordinal scaling.

**17.** Method of claim 16, wherein 3-level ordinal scaling is used, and the values of the matrix were replaced by 0, 1, or 2, depending on whether the value was below, in, or above the range defined by the $x^{th}$ and $y^{th}$ inter-quantile range of a corresponding control group.

**18.** Method of claim 17, wherein x is 10 and y is 90.

**19.** Method of claim 16, wherein binary scaling is used.

**20.** Method of any one of the preceding claims, wherein step c) comprises assigning the individual coded data to groups.

**21.** Method of claim 20, wherein the groups correspond to specific dosing levels of a specific compound.

**22.** Method of claim 20 or 21, wherein in step d) the predictive output is generated for at least one group.

**23.** Method of any one of the preceding claims, wherein the biological characteristic of a substance is its toxicity.

# Fig. 1

| **Sample analysis** | **Data analysis** | | | |
|---|---|---|---|---|
| Measurement and basic data transformation (FID to spectra, m/z records to chromatograms and mass spectra) | Data pre-processing = data coding | | | Numerical analysis |
| Delivery of primary data → | Alignment of signals or binning | Normalize data sample wise to unit integral = convert primary data into profiles | Replace missing values by median of reference values, re-code data into deviation profiles with ordinal scale **= scaling by IQRHILO coding** | Model and classify all profiles coded as deviation profiles, predict from deviation profile type |
| Assure analytical quality | Assure equity of recorded signals | Eliminate global elements of confounding variability (shared variability affecting all samples | Isolate significant treatment related differences between groups (samples) of interest, eliminate confounding individual or time dependent parts of normal variability | Assign meaning to the changes, interpret in the context of the original research question |

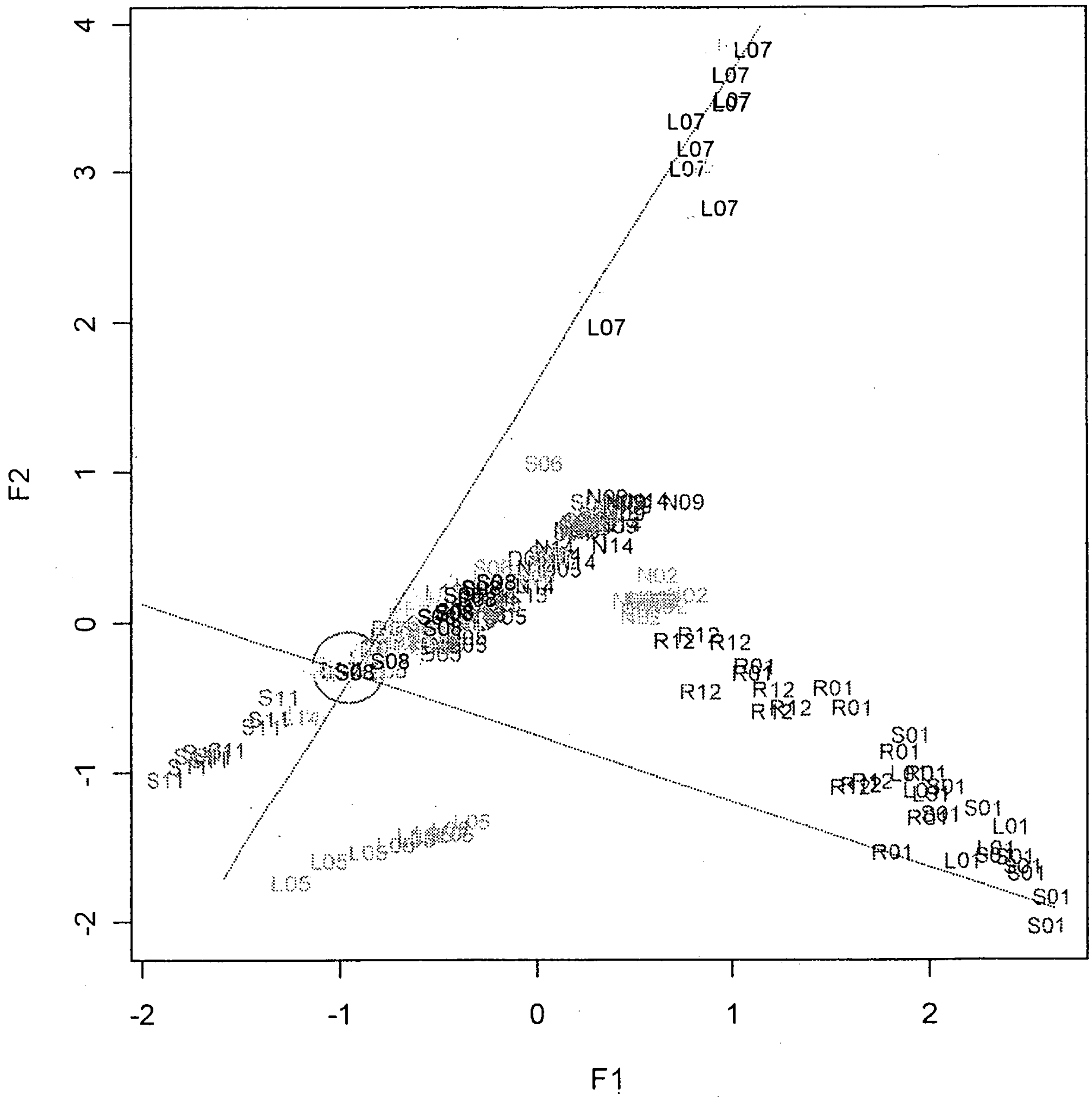
F2
F1
4
3
2
1
0
-1
-2
-2
-1
0
1
2
L07
S06
N09
N14
N02
R12
R01
S08
S11
L05
S01
L01

**Fig. 2A**

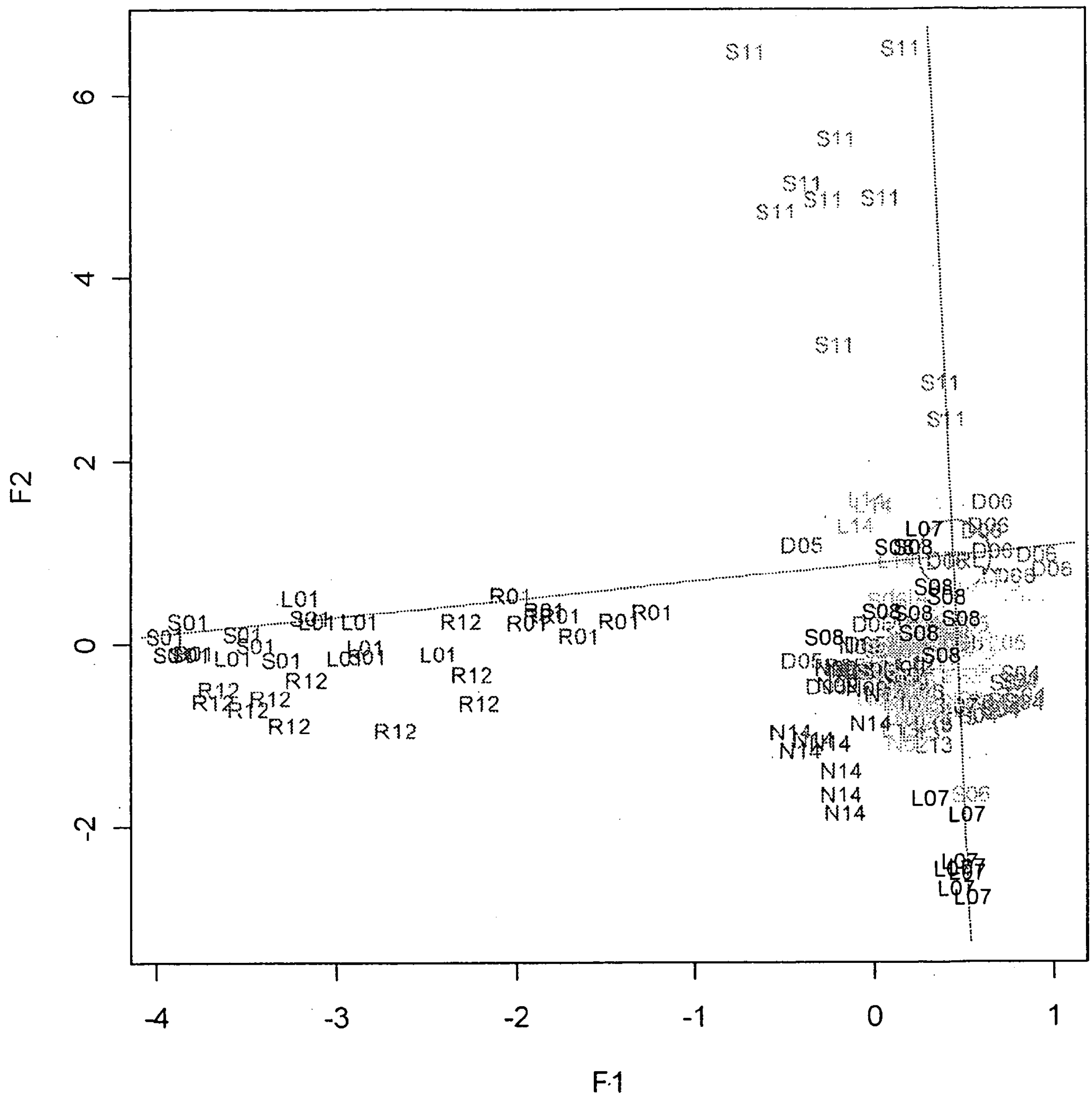


**Fig. 2B**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **LINDON, J.C. et al.** Contemporary issues in toxicology - The role of metabonomics in toxicology and its evaluation by the COMET project. *Toxicology and Applied Pharmacology,* 2003, vol. 187, 137-146
- **EBBELS, T. M. ; H. KEUN et al.** Toxicitiy classification from metabonomic data using a density superposition approach: ''CLOUDS. *Analytica Chimica Acta,* 2003, vol. 490, 109-122
- **VENABLES, W.N. ; RIPLEY, B.D.** Modern Applied Statistics with S. Springer, 2002